(19)

Europäisches
Patentamt
European
Patent Office
Office européen
des brevets

(11) **EP 4 179 964 A1**

(12) **EUROPEAN PATENT APPLICATION**
published in accordance with Art. 153(4) EPC

(43) Date of publication:
**17.05.2023 Bulletin 2023/20**

(21) Application number: **21842336.6**

(22) Date of filing: **13.07.2021**

(51) International Patent Classification (IPC):
**A61B 5/01** (2006.01)    **A61B 5/00** (2006.01)

(86) International application number:
**PCT/CN2021/106121**

(87) International publication number:
**WO 2022/012557 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.07.2020 CN 202010672275**

(71) Applicant: **Wei, Qiang**
**Zhengzhou, Henan 450004 (CN)**

(72) Inventor: **Wei, Qiang**
**Zhengzhou, Henan 450004 (CN)**

(74) Representative: **Gulde & Partner**
**Patent- und Rechtsanwaltskanzlei mbB**
**Wallstraße 58/59**
**10179 Berlin (DE)**

(54) **METHODS AND SYSTEMS FOR MEASURING STRENGTH AND VARIATION OF NUTRITIVE QI AND DEFENSIVE QI**

(57) This application disclose a method and system for measuring the strength and variation of Ying-nutrient Qi & Wei-defensive Qi by measuring infrared radiation and body surface temperature: heating tissue to a set temperature on a site near a human meridian, and then measuring infrared radiation to calculate an obscuration ratio so that a numerical representation of the strength of Ying-nutrient Qi can be obtained. By measuring the strength of the Ying-nutrient Qi in multiple meridians at the same time, and comparing multiple results obtained to obtain the relative strength of the Ying-nutrient Qi of different meridians. A Ying-nutrient Qi measurement system, comprising at least two pairs of infrared temperature sensors and contact temperature sensors, a CPU, and a storage device, wherein the contact temperature sensors are in direct contact with a human body, and every infrared temperature sensor is paired with a contact temperature sensor to measure the temperature of a site by using the method and system of the application, whether a patient has blood stasis or phlegm retention can be inferred. In addition, a fluctuation condition of Ying-nutrient Qi or the relative strength of Ying-nutrient Qi/Wei-defensive Qi of different meridians can further provide auxiliary bases for syndrome differentiation of six meridians.

FIG.5

## Description

## Background

The present application relates to the field of traditional Chinese medicine, and in particular to methods and systems for measuring strengths and variations of Ying-nutrient Qi and Wei-defensive Qi in meridians by measuring infrared thermal radiation and body surface temperature.

Over the years, people have conducted extensive and in-depth research on meridians including research on meridians themselves and using meridian information to help with diagnosis. The methods used include the resistance/conductivity method, high-frequency vibroacoustic method, spectroscopy method, laser intensity method, isotope tracer method and so on. However, these methods have corresponding disadvantages. For example, the measurement values obtained by the resistance/conductance method are unstable, the high-frequency vibroacoustic method will damage the meridians, and the measurement values got by the spectral method/laser intensity method cannot be used to distinguish normal people and patients, etc. In addition, most existing research methods regard meridian Qi and blood as a whole without distinguishing between Ying-nutrient Qi and Wei-defensiveQi, which leads to the measurement results (such as the measurement results obtained by the resistance/conductivity method) being affected by both, and thus, resulting in confusion.

Meridian research using body surface temperature or infrared thermal imaging cameras is another important branch, and many achievements have been obtained so far. For example, Katsusuke Serizawa and Yu Fenglan found that the temperature of acupoints is generally higher than that of non-acupoints. Xu Jinsen, Hu Xianglong et al. obtained the high-temperature lines along the meridians by using infrared thermography after heating the acupoints and proved the existence of meridians from a new perspective. Zhang Dong, Liu Ruiting, and others found that piercing Hegu would cause the temperature of Yingxiang point to rise. In contrast, Ma Huimin and others found that after piercing Quchi, the infrared temperature of Quchi, Wenliu, and Shousanli would fall. However, these achievements cannot be used to help with diagnosis. In addition, later studies generally use infrared thermal imaging cameras. These studies, while overlooking the influence of emissivity, believe that infrared temperature is an accurate representation of temperature. However, according to the method of this invention, it can be seen that the intensity of infrared radiation on the meridians and acupuncture points of the human body changes with the seepage of Ying-nutrient Qi (if the concept of emissivity is mechanically applied, the emissivity will change). So, the infrared thermal imager can only obtain approximate information and may not accurately reflect the condition of the human body. In addition, it is difficult for thermal imaging cameras to give a curve of the temperature of a part over time, which can provide a lot of information.

Another approach is to measure the temperature of acupoints to measure the strength of meridian Qi/blood or to know whether the person is sick (help with diagnosis). Ogihara found that the patient will feel uncomfortable if the temperature difference between the left and right of the same acupoint exceeds 0.5 degrees. Zhao Rongzhu, Gao Yanbin, etc., found that the temperature difference between the left and right acupoints of gastritis and diabetic patients is higher than that of healthy people. Experiments by Li Zishuang and Lin Huilan found that the temperature in Ganshu and Taichong in patients with liver disease is higher than that in healthy people. At present, the main idea of auxiliary diagnosis is to use thermal resistance or thermocouple to measure the skin surface temperature or to use an infrared thermal imager to measure the temperature and then compare the temperature of the same acupoint on the left and right sides. If the temperature difference is too large (such as exceeding 0.5 degrees), there is a problem with the corresponding meridians/viscera. However, this evaluation method is too simple and is not easy to be used together with pattern differentiation by the eight principles or pattern differentiation by the six meridians.

## Description of Related Arts

A technical problem to be solved by the present invention is to provide a method and system for measuring the strength and variation of Ying-nutrient Qi in meridians by measuring infrared thermal radiation and body surface temperature at the same time, and a method and system for measuring the strength of Wei-defensive Qi in meridians. Although neither information on whether a human is healthy (the vast majority of people are in a sub-health state, with dual deficiency of Qi and blood) nor disease diagnosis results can be obtained directly by these methods and systems, they can provide more supplementary (intermediate) information for user reference.

## Summary

A method for measuring the strength of Ying-nutrient Qi, comprising: selecting a site on a running path of a meridian in a human body, heating the surrounding tissues to a set temperature, and then measuring infrared thermal radiation at the site to calculate an obscuration ratio, thereby obtaining a numeralized representation of the strength of the Ying-nutrient Qi.

According to an embodiment of the present application, furthermore: measuring the strengths of the Ying-nutrient Qi in multiple meridians in a human body at the same time and then comparing the multiple results obtained to obtain the relative strength of the Ying-nutrient Qiof different meridians.

A method for measuring the strength of Ying-nutrient Qi, comprising: measuring a temperature at a certain depth under the skin with a penetrating temperature sensor near a running path of a meridian in a human body, and then measuring infrared thermal radiation at the place to calculate an obscuration ratio, thereby obtaining a numeralized representation of the strength of the Ying-nutrient Qi.

A method for measuring the strength of Wei-defensive Qi, comprising: attaching a to-be-heated medium covered with a thermal insulating layer outside to a site on a running path of a meridian in a human body, recording a temperature-rising curve of the medium, and then calculating a temperature-rising rate for evaluating the strength of the Wei-defensive Qi at the corresponding site.

According to an embodiment of the present invention, the method further includes: measuring the strengths of the Wei-defensive Qi in multiple meridians in a human body at the same time, and then comparing multiple results obtained to obtain the relative strength of the Wei-defensive Qi of different meridians.

A system for measuring the strength and variation of Ying-nutrient Qi in a human body, comprising: at least two pairs of infrared temperature sensors and contact temperature sensors, a CPU, and a storage device, wherein the contact temperature sensors are in direct contact with a human body, and every infrared temperature sensor is paired with a contact temperature sensor to measure the temperature of a site.

According to an embodiment of the present application, furthermore, the infrared temperature sensors each have a waterproof structure.

According to an embodiment of the present application, furthermore, the storage device stores two types of temperature data and corresponding timestamps at the same time, with a storage duration of greater than 30 minutes.

A system for measuring the strength of Wei-defensive Qi, comprising: a CPU, a storage device, at least two to-be-heated mediums capable of contacting and being heated by a human body, and corresponding temperature sensors placed within the mediums, wherein the CPU records a temperature-rising curve of the to-be-heated medium, and calculates a temperature-rising rate for evaluating the strength of the Wei-defensive Qi at the corresponding site.

According to an embodiment of the present application, furthermore, the to-be-heated medium is covered with a thermal insulating layer.

Beneficial Effects

According to the methods and systems of the present invention, the abrupt changes of Ying-nutrient Qi over time can be seen visually, whereby whether a user is in a Qi-blood stagnation condition such as blood stasis and phlegm-fluid retention can be in turn deduced. It is possible to deduce whether Qi-blood discontinuation occurs based on the obviously abnormal interrelation between infrared radiation and body surface temperature. Furthermore, the syndrome-differentiation of the six meridians can also be provided with auxiliary information by analyzing the fluctuations of Ying-nutrient Qi and comparing the strengths of Ying-nutrient Qi/Wei-defensive Qi in different meridians. Therefore, the present invention represents notable progress and demonstrates practical values.

**Brief Description of the Drawings**

In order to more clearly illustrate the embodiments of the present application, the following will briefly introduce the accompanying drawings that need to be used in the description of the embodiments.

Fig. 1 is an oscillogram obtained by a system for measuring Ying-nutrient Qi according to the present application, showing changes in body surface temperature and no change in infrared radiation;

Fig. 2 is an oscillogram obtained by a system for measuring Ying-nutrient Qi according to the present application, showing abnormality (burrs) caused by Qi-blood stasis in meridians;

Fig. 3 is an oscillogram obtained by a system for measuring Ying-nutrient Qi according to the present application, showing abnormality (reversal) caused by stagnation in meridians;

Fig. 4 is diagram of a human body surface hierarchical model for understanding the present application;

Fig. 5 is a structural diagram of a system for measuring Ying-nutrient Qi according to the present application;

Fig. 6 is a structural diagram of a system for measuring Wei-defensive Qi according to the present application;

Fig. 7 is a structural diagram of a to-be-heated medium used in a system for measuring Wei-defensive Qi, and an accessory structure thereof;

Fig. 8 is a schematic diagram of a temperature-rising curve obtained by using a system for measuring Wei-defensive Qi according to the present application.

**Detailed Description of the Embodiments**

The following will clearly and completely describe the embodiments of the present application with reference to the accompanying drawings. Obviously, the described embodiments are only some, not all, embodiments of the present application. Based on the embodiments of the present application, all other embodiments obtained by persons of ordinary skill in the art without making creative efforts belong to the protection scope of the present application.

Before explaining the specific implementation, three basic principles used in the present application are firstly explained: First, the Qi running inside and outside the meridian includes Ying-nutrient Qi and Wei-defensive Qi. That is to say, the channel in "the Ying-nutrient Qi runs inside the channel, and Wei-defensive Qi outside the channel" are meridians rather than blood vessels. Second, there will be a small amount of seeping while the Ying-nutrient Qi runs in meridians. The more prosperous the Ying-nutrient Qi, the more seepage (but even more seeping out when the meridians are blocked). When the Ying-nutrient Qi seeps out, it will generate heat, and the product will affect (shade) the infrared radiation. Third, the Wei-defensive Qi outside the meridians has the function of keeping warm. If the Wei-defensive Qi is strong, the heat dissipation through convection and conduction will be slow, and the temperature of the corresponding parts of a human body (especially the internal temperature) will be higher. However, the Wei-defensive Qi basically does not affect the transmission of infrared rays.

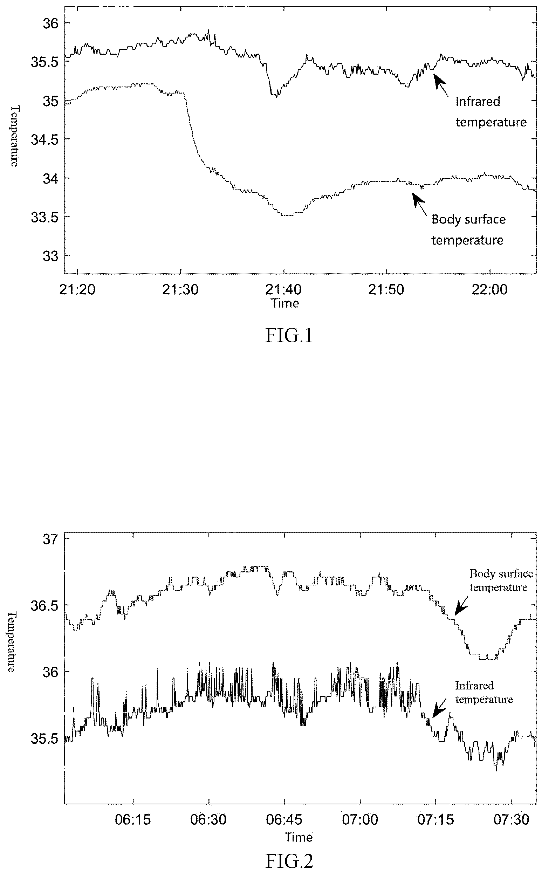

The following analysis (materials) are provided for the establishment of these three principles: First, according to an implementation method of the present application, when measured in a human body(especially at the beginning of traditional twelve two-hour periods), there will often be body surface temperature changes, but the infrared radiation remains constant at the time(Fig. 1). Wei-defensive Qi can well explain this phenomenon. That is, the heat production and internal temperature of the human body remain unchanged at this time, and Wei-defensive Qi does not block infrared rays, so the infrared radiation remains unchanged. However, the change of Wei-defensive Qi leads to the change of heat dissipation rate, so the body surface temperature changes accordingly.

Secondly, the waveform in Fig. 2 can often be seen for patients with blood stasis or phlegm-fluid retention --- the infrared radiation presents sharp changes (burrs). However, the patient's body surface temperature does not change much. When measuring on hands, it often happens that waveforms measured on some fingers have burrs, but waveformsmeasured on adjacent fingers have not, which can rule out changes in infrared radiation caused by changes in blood flow velocity. This waveform cannot be explained by Wei-defensive Qi but can only be explained by introducing the mechanism that Ying-nutrient Qi generates heat. That is to say, for these patients, the flow of Ying-nutrient Qi is no longer smooth but intermittent, and the Ying-nutrient Qi also leaks intermittently to generate heat, and a sudden change in infrared radiation will appear. The explanation for the heat generated by Ying-nutrient Qiseepage is also consistent with the current research results that the temperature along the meridians is higher than that around the meridians.

Thirdly, according to the explanation of Ying-nutrient Qi seeping generate heat, the temperature on the upstream part of the meridian (for example, Hegu is on the upstream part when needling in Quchi) should rise when needling (because the flow of Ying-nutrient Qi is blocked). However, existing studies have shown that infrared radiation (infrared temperature) on the upstream part of the meridian (against the running direction of the Ying-nutrient Qi) will decrease during acupuncture. Therefore, the most reasonable explanation is that seeping and heating products of Ying-nutrient Qi will block infrared radiation.

The test carried out according to the implementation method of the present application also shows that when the temperature of finger tissue is fixed at the temperature of the constant temperature water tank, the infrared radiation of healthy people with strong Qi is lower when compared with people with weak Qi (the difference between infrared radiation temperature and the temperature of the constant temperature water tank is larger). This phenomenon also confirms that Ying-nutrient Qi seeps out and shields infrared radiation.

Finally, if one agrees with the above inference, one must also agree that Ying-nutrient Qi and Wei-defensive Qi are running inside and outside the meridians simultaneously, and the channels in "the Ying-nutrient Qi runs inside the channel, and Wei-defensive Qi outside the channel" are meridians rather than blood vessels.

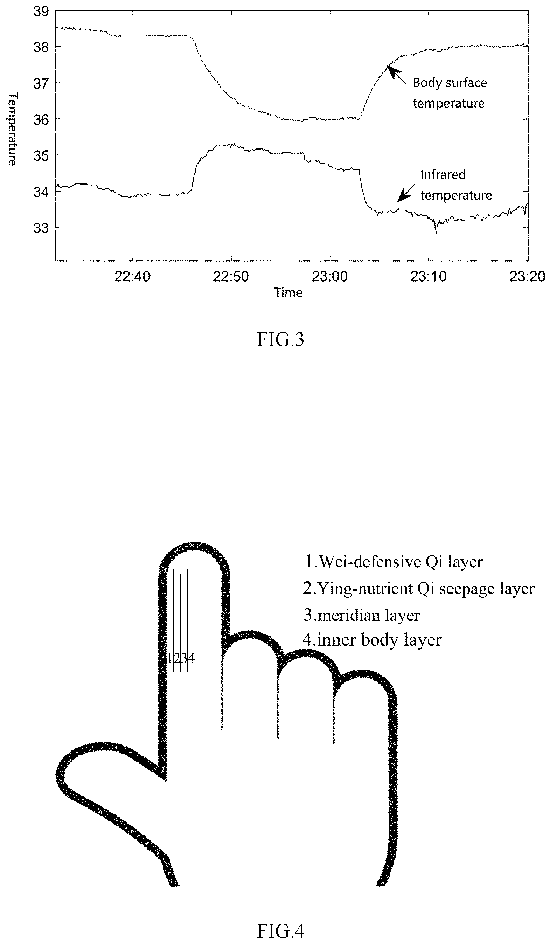

According to the principles mentioned above, the meridian model shown in Fig. 4 can be constructed. The model includes the inner body layer, meridian layer, Ying-nutrient Qiseepage layer, Wei-defensiveQi layer, and epidermis (Ying-nutrient Qiseepage layer and Wei-defensiveQi layer are actually integrated rather than distinct). In the model, the inner layer is the heat source, and the heat in the limbs mainly comes from tissues such as blood and muscles (but also from the seepage layer of Ying-nutrient Qi). The meridian layer is a thin line with a diameter of one millimeter or several millimeters, corresponding to the meridians of a human body. The seepage layer will generate heat and increase the temperature of the inner layer, and at the same time, block the infrared rays emitted by the inner layer (of course, this layer itself also emits infrared rays, but its strength is relatively small, so the proportion of these infrared rays that are blocked can be ignored). The Wei-defensiveQi layer plays a role in keeping warm. The stronger the Wei-defensiveQi is, the less heat will be lost through convection (that is the embodiment of the function of the Wei-defensiveQi keeping the body warm), and the corresponding inner layer temperature and body surface temperature will be higher. However, the Wei-defensiveQi layer does not affect the transmission of infrared radiation.

It should be noted that this model is an equivalent model. That is to say, the model is mainly given for easy understanding of subsequent embodiments, and its structure is entirely different from the physiological skin model. Therefore, a layer in this model cannot and should not be equated with a structure in any human tissue.

The methods and systems of the present application are provided below according to the foregoing principles and models.

A method for measuring the strength of Ying-nutrient Qi, comprising: selecting a site on a running path of a meridian in a human body, heating the surrounding tissues to a set temperature, and then measuring infrared thermal radiation at the site to calculate an obscuration ratio, thereby obtaining a numeralized representation of the strength of the Ying-nutrient Qi.

During implementations, heat sources such as infrared lamps cannot be used for heating, otherwise, uneven heating will be caused at different sites in a human body. A recommended method includes immersing hands in a thermostatic water tank. A recommended temperature is 38°C, which is higher than the temperature of the hands and does not lead to sweating which affects the ratio at which infrared rays are obscured. After a temperature field at different sites on hands becomes even by being heated for a period of time (such as 15 minutes), a miniature infrared temperature sensor can be placed on a running path of a meridian in each finger to measure infrared radiation. It can be found from the measurement results that the infrared temperature got by different sensor is different, and is generally lower than 38°C. Here, the following formula may be used to obtain an obscuration ratio:

$$k=(38-T_o)/38=1-T_o/38 \quad (1)$$

k obtained here is a value between 0 and 1, and the larger the value, the stronger the strength of the corresponding Ying-nutrient Qi. Nevertheless, for the sake of simplicity, a temperature difference may also be used directly to evaluate the strength of the Ying-nutrient Qi.

$$\Delta T_o=38-T_o \quad (2)$$

The larger the temperature difference obtained, the stronger the strength of the corresponding Ying-nutrient Qi is. This is also the practice to be used hereinafter. That is, the temperature difference value is used to represent the strength of Qi & blood.

In addition to measurement on fingers, the aforementioned method can also be applied to other parts of limbs. Here, the thermostatic water tank can be changed to other thermostatic heating means capable of covering the sites to be measured. However, this method cannot be applied to the trunk, since it is difficult to ensure constant internal temperature due to many other internal heat sources (internal organs, etc.).

According to an embodiment of the present application, furthermore, the infrared temperature sensors each have a waterproof structure.

There are a variety of measures for achieving the waterproof structure. For example, the housing of each sensor can be sealed with adhesive or a rubber seal ring, etc. Due to the use of the waterproof structure, the infrared temperature sensors can be immersed in water for use. As such, a certain site on a human body, together with the infrared temperature sensor, can be immersed in the thermostatic water tank; after a period of time, the infrared radiation temperature of a site where a meridian passes nearby is measured in the water tank (without taking the measured site out of the water tank); and then, an obscuration coefficient of the seepage of Ying-nutrient Qi can be obtained by using formula (1), whereby the strength of the Ying-nutrient Qi can be evaluated.

Such a practice of measuring the intensity of infrared radiation after heating an object to a preset temperature is seemingly somewhat similar to measuring emissivity. But the two should not be confused. This is because the emissivity is only applicable to an object made of a single material (such as metal, plastic, cement, etc.), while a human body is a complex organism in which infrared radiation is affected by many factors such as the blood flow rate, the strengths of Ying-nutrient Qi and Wei-defensive Qi and sweating. Therefore, the concept of emissivity should not be used here, nor can infrared radiation and body surface temperature be expected to achieve a fixed ratio.

It should be noted that there is an alternative method to achieve the object of this embodiment. For example, in case the infrared temperature sensor may not have a strict waterproof design, a tester can stick the sensor to a finger, then wear a waterproof glove, and immerse the hand into the thermostatic water tank. Such an alternative design is essentially the same as this embodiment, except in that the waterproof structure is expanded to include the hands of a test subject and the sensors.

According to an embodiment of the present application, furthermore: measuring the strengths of the Ying-nutrient Qi in multiple meridians in a human body at the same time and then comparing the multiple results obtained to obtain the relative strength of the Ying-nutrient Qi of different meridians.

During simultaneous measurement and comparison, measuring multiple meridians in multiple fingers is also the most convenient way. The reason lies in that the fingers are anatomically similar, showing good comparability. After the relative strength is obtained, it can be used as intermediate information to assist diagnosis. In fact, a traditional Chinese medicine doctors often makes a prescription based on the relative strength of Ying-nutrient Qi, instead of the absolute strength of the Ying-nutrient Qi (patients with longstanding disease may have weaker Ying-nutrient Qi in all meridians than normal people). For example, if the Ying-nutrient Qi corresponding the liver and kidneys is weaker, targeted treatment can be carried out. However, the strength of Qi and blood in the meridians should not be directly associated here with the name of a disease or a syndrome. The connection must be made by a doctor by using various other symptoms as well.

The thermostatic method as mentioned above cannot be used for sites on the trunk and the like, and an alternative method can be used.

A method for measuring the strength of Ying-nutrient Qi, comprising: measuring a temperature at a certain depth under the skin with a penetrating temperature sensor near a running path of a meridian in a human body, and then measuring infrared thermal radiation at the place to calculate an obscuration ratio, thereby obtaining a numeralized representation of the strength of the Ying-nutrient Qi.

According to an embodiment of the present application, a temperature measured deep beneath the human skin (acupuncture and moxibustion textbooks will give different penetration depths for various acupoints, this depth can be used when measuring) by a penetrating temperature sensor (for example, a thermal resistor may be installed at the tip of an acupuncture needle) can be used to replace the constant temperature of 38°C in the formula (1), and the infrared temperature measured at the same site is then brought into the formula, whereby the obscuration coefficient k of a seepage layer of Ying-nutrient Qi may also be obtained. During implementation, it should be noted that a penetration position should keep away from but approach the meridians as much as possible. The reason for this is that the seepage of Ying-nutrient Qi is not limited to regions directly above the meridians, but spreads nearby. Therefore, a near-region measurement may have a similar effect. At the same time, keeping away from the meridians can prevent damage caused by blocking the Qi-blood circulation. However, this method is invasive and may also disrupt the Qi-blood circulation and cause possible damage if the meridians are pierced accidentally. Therefore, this is not a recommended practice.

A method for measuring the strength of Wei-defensive Qi, comprising: attaching a to-be-heated medium covered with a thermal insulating layer outside to a site on a running path of a meridian in a human body, recording a temperature-rising curve of the medium, and then calculating a temperature-rising rate for evaluating the strength of the Wei-defensive Qi at the corresponding site.

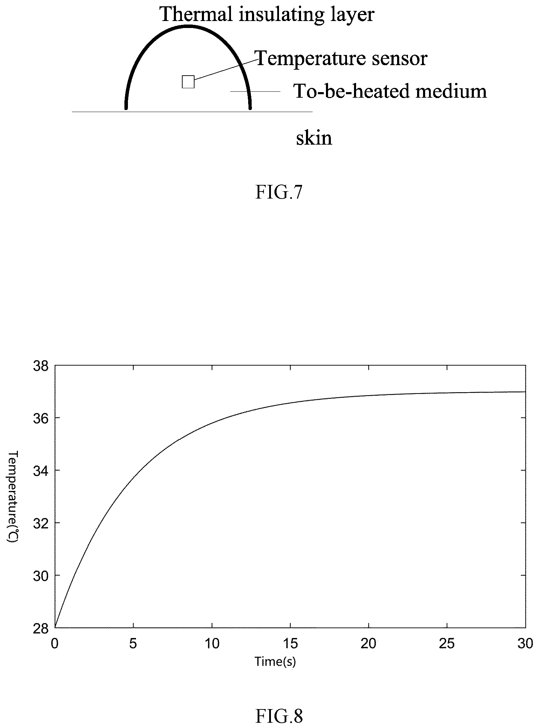

According to an embodiment of the present application, water and Resistance Temperature Detector(RTD)/thermocouple temperature sensors can be placed in a double-layer hemispherical structure with a vacuum in the middle (this structure is actually a common structure in daily life, such as a double-layer stainless steel bowl or a thermos cup). Before the test starts, use a constant temperature water tank to ensure that the water is at a certain temperature (such as 28 degrees). At the beginning of the test, the structure is covered on the tested part, and the human body will simultaneously heat the water through convection/radiation/conduction. The recorded temperature profile will resemble the charging profile of an RC circuit (Fig. 8). At this time, the strength of Wei-defensive Qi can be measured by calculating the heating rate (for example, the rising slope in the first 10 seconds). The faster the heating rate, the weaker the Wei-defensive Qi.

It should be noted that there are many other indicators to represent the strength of Wei-defensive Qi at this time. For example, assuming that the final value of the heating curve is Tf, the time to reach the temperature of 0.63 (Tf-28) (similar to RC charging time) can also be used as an indicator. The shorter the time, the weaker the Wei-defensive Qi. Of course, this is not the only indicator that can be used. Other indicators can also be calculated by using the temperature rise curve to evaluate the strength of Wei-defensive Qi.

According to an embodiment of the present application, metal can also be selected as the to-be-heated medium. At this time, different heat transfer speeds caused by different convective speeds (caused by different placement directions of the medium) will not be a problem, and the measurement results will be more accurate.

When performing the aforementioned measurements, it should also be known that Wei-defensive Qi often undergoes a sudden change at the junction of different traditional twelve two-hour periods, so the measurement should not be done during the junction time(unless one wants to know the sudden change of Wei-defensive Qi caused by the midnight-noon ebb-flow), and the measurement results must be used when the influence of measurement time is considered.

However, because a human body is a very complex organism, many factors are difficult to control precisely (such as food intake, mood, etc.). Therefore, even under strict test conditions, the heating rate obtained by this method may vary in a wide range. In other words, it is difficult to directly compare the strength of Wei-defensive Qi obtained by different people at different times. Therefore, the solution is to compare the heating rate values of different meridians of the same individual at the same time to obtain the relative strength of Wei-defensive Qi in different meridians.

According to an embodiment of the present application, furthermore: measuring the strengths of the Wei-defensive Qi in multiple meridians in a human body at the same time, and then comparing multiple results obtained to obtain the relative strength of the Wei-defensive Qi of different meridians.

When measuring simultaneously and comparing, measuring multiple meridians at multiple fingers is convenient. Because the anatomical structures of multiple fingers are similar, the comparability is good. After obtaining the relative strength, it can be used for auxiliary diagnosis and treatment. Generally speaking, when Chinese medicine is prescribed, it is often not based on the absolute strength of Wei-defensiveQi (prolonged illness patients may have weaker Wei-defensiveQi in all meridians than healthy people) but relative strength. Note, however, that the strength of Qi& blood in any meridian should not be directly related to the name of a disease or a syndrome. The doctor must combine various other symptoms to make a judgment.

A system for measuring the strength and variation of Ying-nutrient Qi in a human body, comprising: at least two pairs of infrared temperature sensors and contact temperature sensors, a CPU, and a storage device, wherein the contact temperature sensors are in direct contact with a human body, andevery infrared temperature sensor is paired with a contact temperature sensor to measure the temperature of a site.

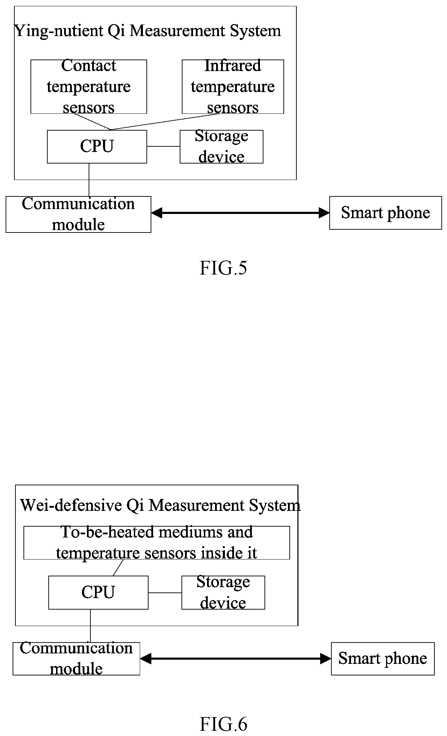

Fig. 5 is a diagram showing a structure of the system, in which a CPU, a storage device, and sensors are included. The CPU acquires the measurement results of the infrared temperature To and body surface temperature Ta from the sensors by means of communication or A/D conversion, and stores these results in the storage device. When a user makes a query, the CPU acquires the current values or historical curves of the infrared temperature and body surface temperature from the storage device, and provides them to the user in pairs simultaneously.

If a human body undergoes Qi-blood stagnation (such as disharmony between the heart and the kidney, or cold limbs) in meridians, the upstream meridians may demonstrate a significantly low infrared temperature and significantly high body surface temperature for a long time (the $\Delta Ta=To-Ta$ corresponding to a certain meridian will be significantly different from other meridians). Because this is a qualitative change with relatively obvious characteristics, it can be visually observed using the present system. However, since this method requires comparison among multiple meridians, the system should provide multiple pairs of sensors.

Because the infrared temperature (due to obscuration) is lower when more Ying-nutrient Qi seeps, and the body surface temperature is higher due to heating caused by the seepage of Ying-nutrient Qi, if the strength of Wei-defensive Qi of each meridian is considered to be relative equilibrium, the difference value $\Delta Ta=To-Ta$ between the infrared temperature To and the body surface temperature Ta may approximately represent the strength of the Ying-nutrient Qi (in particular the relative strength among multiple meridians). In general, the stronger the Ying-nutrient Qi, the smaller the corresponding $\Delta Ta$ (this rule still holds in some cases where the measured infrared temperature is lower than the body surface temperature). Note that, since the body surface temperature and $\Delta Ta$ are also significantly affected by the strength of the Wei-defensive Qi, and the Wei-defensive Qi often does not follow its path, this rule should be applied with care. However, coincidentally, the deficiency of Wei-defensive Qi will also reduce the body surface temperature and increase the value of $\Delta Ta$ in most cases. Consequently, if the value of $\Delta Ta$ in a certain meridian is significantly greater than that in other meridians, weak Ying-nutrient Qi or weak Wei-defensive Qi can be suspected. Since the thermostatic water bank and other tedious steps can be omitted, the indicator $\Delta Ta$ can be used for preliminary screening or approximate judgment.

According to an embodiment of the present application, the contact temperature sensor may also be a penetrating temperature sensor (for example, a thermal resistor may be installed on the tip of an acupuncture needle). That is, a temperature measured deep beneath the human skin (acupuncture and moxibustion textbooks will give different penetration depths for various acupoints, this depth can be used when measuring) can be used to replace the constant temperature of 38°C in the formula (1), and then the infrared temperature measured at the same site is then brought into the formula, whereby the obscuration coefficient k of a seepage layer of Ying-nutrient Qi may be obtained. Based on this, the strength of the Ying-nutrient Qi can be evaluated.

According to an embodiment of the present application, furthermore, the infrared temperature sensors each have a waterproof structure.

There are many measures to achieve a waterproof structure. For example, the housings of sensors and electronic components can be sealed with waterproof paint or glue, or rubber seal rings, etc.Due to the use of the waterproof structure, the infrared temperature sensors can be immersed in water for use. As such, a certain part of a human body can be immersed in a thermostatic water tank (the recommended temperature is 38°C); after a period of time, the infrared radiation temperature of a site where a meridian pass nearby is measured in the water tank; and then, an obscuration coefficient indicative of the seepage of Ying-nutrient Qi can be obtained, whereby the strength of the Ying-nutrient Qi can be evaluated.

It should be noted that there is an alternative method to achieve the object of this embodiment. For example, in case the infrared temperature sensor may not have a strict waterproof design, a tester can stick the infrared sensor to a finger, then wear a waterproof glove, and immerse the hand into the thermostatic water tank. Such an alternative design is essentially the same as this embodiment, except in that the waterproof structure is expanded to include the hands of a test subject and the sensors.

Because a human body is a very complex organism in which many parameters cannot be accurately controlled, the absolute value of the strength of Ying-nutrient Qi measured by the aforementioned process can hardly be compared with a standard value, and it is more meaningful to measure the relative strength of multiple meridians. Therefore, the system should provide multiple pairs of infrared temperature sensors and contact temperature sensors simultaneously.

According to an embodiment of the present application, furthermore, the storage device stores two types of temperature data and corresponding timestamps at the same time, with a storage duration of greater than 30 minutes.

After long-term storage of temperature trends, the system may be used to monitor the abrupt changes and periodical fluctuations of the strength of the Ying-nutrient Qi. As described above, if a user has problems such as blood stasis or phlegm-fluid retention, the Qi-blood circulation will be suddenly blocked and then dredged. At this time, burrs will occur to the historical curves of the infrared temperature. Looking at the current temperature reading alone will not reveal a problem at this time, but the trend graph will provide obvious information. Furthermore, some patients (with a problem in shaoyang meridians, for example) when measured show repeatedly fluctuating infrared temperature and body surface temperature. As such, the value of infrared/body surface temperature at a certain time should not be used alone. That is to say, the trend chart over a period of time should be used here. Therefore, the storage device should be capable of storing historical information for a sufficiently long time (at least 5 minutes, and more than half an hour as recommended).

According to an embodiment of the present application, furthermore, the contact temperature sensors are integrated in the infrared temperature sensors. Melexis's 90615 and 90632 both provide such a structure. When 90615 and 90632 are put on the skin, an object temperature To measured is the infrared temperature, and an ambient temperature Ta obtained is the body surface temperature. If 90615 is used, it should be noted that unreasonable infrared data may appear during the transition of a body surface/ambient temperature change. This is because the temperature field inside 90615 haven't become even. Therefore, these unreasonable data should be excluded with care to avoid an erroneous conclusion. Furthermore, a silicone ring with a thickness smaller than that of 90615 may also be used outside 90615, such that the sensor can be prevented from moving while ensuring the reliable contact between 90615 and the skin. Moreover, the CPU and memory of Arduino DUE may be used directly as the CPU and memory device. Here, the CPU communicates with 90615 or 90632 by using the SMBUS protocol, obtains the infrared temperature and body surface temperature simultaneously and store them in the memory.

According to an embodiment of the present application, furthermore, the system may further include a Bluetooth or WIFI communication module for communicating with a smartphone. Since a communication function is achieved, historical records (the trend charts) in the storage device can be uploaded to the mobile phone upon the calling of software in the mobile phone. In such a way, the display/storage/analysis function of the mobile phone can be utilized to significantly prolong the storage time and reduce the volume/cost of a testing apparatus, which is the most preferred embodiment.

According to an embodiment of the present application, connecting lines (communication/power lines) may also be omitted between the CPU and the sensors. At this time, a battery and communication circuits need to be installed in the sensors. In such a way, the volume and cost of the sensors are significantly increased at present, and the recording time is reduced, which are notable disadvantages. But as time goes on and technology improves, these shortcomings may no longer be a problem,

According to an embodiment of the present application, the storage device is a storage device of a smartphone. Here, the CPU directly provides acquired data to the smartphone via communication, and the smartphone is responsible for long-term storage of two types of measured temperature curves and for displaying them to a user.

A system for measuring the strength of Wei-defensive Qi, comprising: a CPU, a storage device, at least two to-be-heated mediums capable of contacting and being heated by a human body, and corresponding temperature sensors placed within the mediums, wherein the CPU records a temperature-rising curve of the to-be-heated medium, and calculates a temperature-rising rate for evaluating the strength of the Wei-defensive Qi at the corresponding site. Fig.6 shows such a system. Fig. 7 is a detailed structural diagram of the to-be-heated medium, temperature sensor, and thermal insulating layer.

According to an embodiment of the present application, the to-be-heated medium may be a small amount of water (or a flowable medium such as alcohol), and a thermal resistance/thermocouple temperature sensor is placed in the water in the structure. Before the test starts, use a constant temperature water tank to ensure that the water is at a specific temperature (such as 28 degrees). At the beginning of the test, the structure is put upon the tested site, and the human body will simultaneously heat the water through convection/radiation/conduction. The recorded temperature curve will be similar to the charging curve of an RC circuit. At this time, the strength of Wei-defensive Qi can be measured by calculating the heating rate (for example, the rising slope in the first 10 seconds). The faster the heating rate, the weaker the Wei-defensive Qi.

It should be noted that there are many other indicators to represent the strength of Wei-defensive Qi at this time. For example, assuming that the final value of the heating curve is Tf, the time to reach the temperature of 0.63 (Tf-28) (similar to RC charging time) can also be used as an indicator. The shorter the time, the weaker the Wei-defensive Qi. Of course, this is not the only indicator that can be used. Other indicators can also be calculated by using the temperature rise curve data to evaluate the strength of Wei-defensive Qi.

According to an embodiment of the present application, the to-be-heated medium can also be metal (such as aluminum, copper, tin, stainless steel or hybrid structure) or other immobile substances, and the thermal resistance/thermocouple temperature sensor is placed in the middle of the to-be-heated medium. The advantage of this method is that the to-be-heated medium does not have internal convection, so the measurement results will not be affected by changes in the heat transfer rate (from the human body to the medium) caused by the sensor placement direction.

According to an embodiment of the present application, furthermore, the to-be-heated medium is covered with a thermal insulating layer.

The thermal insulating layer can use a double-layer hemispherical structure with a vacuum in the middle (this structure is a common structure in daily life, such as double-layer stainless steel bowls or thermos cups). Measurements are more accurate when the to-be-heated medium is covered with thermal insulation.

Since a human body is a very complex organism, many parameters cannot be precisely controlled, it may be difficult to compare the value of the strength of the Wei-defensive Qi obtained by the aforementioned method with a standard value. It is more meaningful to measure the relative strength of different meridian Wei-defensive Qi at the same time. Therefore, the system should provide multiple sets of to-be-heated mediums and temperature sensors at the same time.

According to an embodiment of the present application, furthermore, the system may include a Bluetooth or WIFI communication module for communicating with a smartphone. Since a communication function is achieved, historical records (the trend charts) in the storage device can be uploaded to a mobile phone upon the calling of software in the mobile phone. In such a way, the display/storage/analysis function of the mobile phone can be utilized to significantly prolong the storage time and reduce the volume/cost of a testing apparatus, which is the most preferred embodiment.

According to an embodiment of the present application, connecting lines (communication/power lines) may also be omitted between the CPU and the sensors. Here, a battery and communication circuits need to be installed in the sensors. In such a way, the volume and cost of the sensors are significantly increased at present, and the recording time is reduced, which are notable disadvantages. But as time goes on and technology improves, these shortcomings may no longer be a problem,

According to an embodiment of the present application, the storage device may also be a storage device of a smartphone. Here, the CPU directly provides acquired data to the smartphone via communication, and the smartphone is responsible for long-term storage of two types of measured data and for displaying them to a user.

There are many situations in which the method and system of the present application can be used. For example, when it is suspected that the patient has blood stasis or phlegm-fluid retention, the strength of the Ying-nutrient Qi can be continuously monitored.

When the ambient temperature is precisely controlled, the Ying-nutrient Qi (infrared temperature) in the meridians of healthy people is stable and changes little over time within an hour.

If the corresponding infrared temperature has glitches, as shown in Fig. 2, it can be suspected that the patient has blood stasis or phlegm-fluid retention in the meridians (Fig. 2 shows the observation results on the middle section of the little finger of Hand Shaoyin meridian between 5 am-7 am after the blood stasis happen on the Foot Yangming meridian. Note that because the strength of Qi becomes weaker after 7 am, the burr disappears immediately after 7 am).

However, it should be noted that one must not drink alcohol before the test (otherwise, there will be a similar curve), and even if there is blood stasis/phlegm-fluid retention, it is not always possible to observe glitches everywhere. Because according to midnight-noon ebb-flow (the sequence of Qi flow in meridians), the strength of Qi in different meridians alternately decline and prosper in different traditional twelve two-hour periods. Most of the time, if the place blocked is downstream of the meridian where Qi & blood are flourishing, the clear results can be seen by observing the meridians near the blocked point.

In addition, it should also be pointed out that if the blockage of a meridian is so serious that it is completely blocked, the burr cannot be seen. But if measured at the right time according to the Midnight-noon ebb-flow, it will be observed that the contact temperature (body surface temperature) on some upstream points of the blockage point rises at certain moments while the infrared temperature falls (and vice versa on downstream points). This is because the heat generated by the oozing of Ying-nutrient gas increases significantly. Hence, the internal layer and body surface temperature rise, but the shading coefficient also increases, so the infrared temperature drops. Fig. 3 shows such a measured waveform. The meridian blockage cannot be judged by the infrared temperature change alone. However, if the temperature obtained by the contact temperature sensor is also used, the probability of blockage will be very high when the two change in reverse.

In addition to blood stasis/phlegm retention that can lead to blockage of meridians, other reasons can also cause Qi& blood blockage (such as disharmony between the heart and the kidney or cold limbs). At this time, the changing waveform shown in Fig. 3 may not appear, but the places where upstream meridians run may have a noticeably low infrared temperature and noticeably high body surface temperature for a long time. Therefore, when measuring multiple meridians (especially when measuring on multiple fingers) simultaneously, the manifestation of abnormal meridians will be noticeably different from other meridians, and it is not difficult to make a judgment in conjunction with other symptoms at this time.

Of course, according to traditional Chinese medicine theory, blood stasis or phlegm-fluid retention (or meridian blockage) does not directly correspond to any specific disease (for example, blood stasis may cause insomnia, coronary heart disease, tumor, and many other diseases, but other reasons may also be the cause of these diseases). Moreover, it does not correspond to any specific prescription or treatment method (for example, sometimes Xuefu Zhuyu Decoction should be used to cure blood stasis, but sometimes Guizhi Fuling Pills should be used). Therefore, although the method and system of the present application can give valuable hints, it is neither a diagnosis method nor a treatment method for diseases.

In addition to judging the blockage of meridians, the method and system of the present application can also be used to assist pattern differentiation by the six meridians.

Pattern differentiation of the six meridians is a diagnosis and treatment system proposed by medical sage Zhang Zhongjing. Using the six meridians to differentiate syndromes can get better result with less effort. The corresponding classical formulas prescribed are often effective soon after the patient takes medicine.

However, the current method classifies a patient into a certain syndrome in accordance with the clustering of multiple symptoms. For example, sweating more may be a sign of Taiyang Meridian disease or Yangming Meridian disease, but combined with a floating pulse, there is a high probability that the patient gets Taiyang Meridian disease; if combined with thirst, there is a high probability that the patient gets Yangming Meridian disease.

In other words, the relationship between symptoms and six meridian diseases is not one-to-one but a many-to-many relationship. There cannot be decoupling between symptoms and disease pattern differentiation (not a specific disease but six disease pattern differentiation such as Taiyang disease and Shaoyang disease). Although "Treatise on the Six Seasonal Phases and the Visceral Picture" states that "For pulse strength in carotid: grade 1 means disease in Shaoyang meridian, grade 2 means disease in the Taiayng meridian, grade 3 in Yangming meridian, and grade 4 or more diseases means excessive yin rejecting yang. For pulse strength in the Cunkou area: grade 1 means disease in the Jueyin meridian, grade 2 means disease in the Shaoyin meridian, grade 3 means disease in the Taiyin meridian, grade 4 means blocked yin", but it is challenging to apply.

Using the method and system of the present application, one can intuitively see the strength and change of the Ying-nutrient Qi/Wei-defensiveQi of the six meridians, and this can achieve decoupling. That is, it can help the doctors using the classic formulas to classify the diseases into one of the pattern differentiation of six meridians (or its combination). For example, under the premise of precisely controlling the ambient temperature, if a doctor sees constant fluctuations of the strength of Ying-nutrient Qi (infrared temperature) in a patient's Hand Shaoyang meridian, he can guess that it is a Shaoyang meridian disease. Because the Shaoyang meridian is a hub, Qi& blood will flow back and forth between the Taiyang meridian and Yangming meridian continuously. Similarly, if a doctor sees that Ying-nutrient Qi/Wei-defensiveQi in Yangming meridian is very strong, namely, obviously stronger than other meridians, he can guess it is a Yangming meridian disease. This approach can provide specific auxiliary information in addition to a large number of uncoupled information, so it has a unique value.

When assisting diagnose the pattern differentiation by the six meridians, it should also be noted that a human body's Ying-nutrient Qi and Wei-nutrient Qi fluctuate continuously throughout the day (but are nearly stable within an hour). If a doctor wants to determine the pattern differentiation by the six meridians, he should also consider the time when the disease is about to be relieved (and is about to worsen), which is described in the Shang-HanLun, and measure at the appropriate time. He should not expect clear results if the patient is measured at some random time.

In addition, it should be noted that the pattern differentiation by the six meridians obtained (by using the method and the system of the present application) does not directly correspond to any specific disease, nor does it correspond to any specific prescription or treatment method. Therefore, although the method and system of the present application can give valuable hints, it is neither a diagnosis method nor a treatment method for diseases.

## Claims

1. A method for measuring the strength of Ying-nutrient Qi, comprising: selecting a site on a running path of a meridian in a human body, heating the surrounding tissues to a set temperature, and then measuring infrared thermal radiation at the site to calculate anobscuration ratio, thereby obtaining a numeralized representation of the strength of the Ying-nutrient Qi.

2. The method according to Claim 1, further comprising: measuring the strengths of the Ying-nutrient Qi in multiple meridians in a human body at the same time, and then comparing the multiple results obtained to obtain the relative strength of the Ying-nutrient Qi of different meridians.

3. A method for measuring the strength of Ying-nutrient Qi, comprising: measuring a temperature at a certain depth under the skin with a penetrating temperature sensor near a running path of a meridian in a human body, and then measuring infrared thermal radiation at the place to calculate an obscuration ratio, thereby obtaining a numeralized representation of the strength of the Ying-nutrient Qi.

4. A method for measuring the strength of Wei-defensive Qi, comprising: attaching a to-be-heated medium covered with a thermal insulating layer outside to a site on a running path of a meridian in a human body, recording a temperature-rising curve of the medium, and then calculating a temperature-rising rate for evaluating the strength of the Wei-defensive Qi at the corresponding site.

5. The method according to Claim 4, further comprising: measuring the strengths of the Wei-defensive Qi in multiple meridians in a human body at the same time, and then comparing multiple results obtained to obtain the relative strength of the Wei-defensive Qi of different meridians.

6. A system for measuring the strength and variation of Ying-nutrient Qi in a human body, comprising: at least two pairs of infrared temperature sensors and contact temperature sensors, a CPU, and a storage device, wherein the contact temperature sensors are in direct contact with a human body, and every infrared temperature sensor is paired with a contact temperature sensor to measure the temperature of a site.

7. The system according to Claim 6, wherein the infrared temperature sensors each have a waterproof structure.

8. The system according to Claim 6, wherein the storage device stores two types of temperature data and corresponding timestamps at the same time, with a storage duration of greater than 30 minutes.

9. A system for measuring the strength of Wei-defensive Qi, comprising: a CPU, a storage device, at least two to-be-heated mediums capable of contacting and being heated by a human body, and corresponding temperature sensors placed within the mediums, wherein the CPU records a temperature-rising curve of the to-be-heated medium, and calculates a temperature-rising rate for evaluating the strength of the Wei-defensive Qi at the corresponding site.

10. The system according to Claim 9, wherein the to-be-heated medium is covered with a thermal insulating layer.

FIG.1

FIG.2

FIG.3

1.Wei-defensive Qi layer
2.Ying-nutrient Qi seepage layer
3.meridian layer
4.inner body layer

FIG.4

Ying-nutient Qi Measurement System

| Contact temperature sensors | Infrared temperature sensors |
|---|---|

CPU — Storage device

Communication module ◄────────► Smart phone

FIG.5

Wei-defensive Qi Measurement System

To-be-heated mediums and temperature sensors inside it

CPU — Storage device

Communication module ◄────────► Smart phone

FIG.6

Thermal insulating layer

Temperature sensor

To-be-heated medium

skin

FIG.7

FIG.8

## INTERNATIONAL SEARCH REPORT

| International application No. |
| --- |
| **PCT/CN2021/106121** |

**A.    CLASSIFICATION OF SUBJECT MATTER**

A61B 5/01(2006.01)i;  A61B 5/00(2006.01)i

According to International Patent Classification (IPC) or to both national classification and IPC

**B.    FIELDS SEARCHED**

Minimum documentation searched (classification system followed by classification symbols)

A61B 5

Documentation searched other than minimum documentation to the extent that such documents are included in the fields searched

Electronic data base consulted during the international search (name of data base and, where practicable, search terms used)

CNTXT, CNABS, VEN, OETXT, CNKI: 穴位, 经络, 营气, 卫气, 气血, 温度, 差, 红外, 贴, 皮肤, 表皮, 体表, 检, 测, 传感器, acupoint, temperature, thermal, infrared, skin, sensor

**C.    DOCUMENTS CONSIDERED TO BE RELEVANT**

| Category* | Citation of document, with indication, where appropriate, of the relevant passages | Relevant to claim No. |
| --- | --- | --- |
| Y | CN 105342565 A (XIAOHONGXIANG MEDICAL TECHNOLOGY CO., LTD.) 24 February 2016 (2016-02-24) description, paragraphs [0011]-[0028] | 1, 2 |
| Y | CN 108652595 A (HEALTHENGINE (BEIJING) MEDICAL TECHNOLOGY CO., LTD.) 16 October 2018 (2018-10-16) description, paragraphs [0042]-[0050] | 1, 2 |
| Y | CN 111265405 A (ZHAO, Hongjie) 12 June 2020 (2020-06-12) description, paragraphs [0009]-[0021] | 4, 5, 9, 10 |
| Y | CN 108871609 A (VIVALNK INC SHI WEI ZHANG HUAQUAN XU ZUOFU) 23 November 2018 (2018-11-23) description, paragraphs [0035]-[0064], and figures 1-6 | 4, 5, 9, 10 |
| PX | CN 111887808 A (WEI, Qiang) 06 November 2020 (2020-11-06) claims 1-10 | 1-10 |
| A | WO 2009158161 A2 (YUAN YUN-E et al.) 30 December 2009 (2009-12-30) entire document | 1-10 |

☑ Further documents are listed in the continuation of Box C.    ☑ See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **11 October 2021** | **18 October 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

# EP 4 179 964 A1

<table>
<tr><td colspan="3"><b>INTERNATIONAL SEARCH REPORT</b></td><td colspan="2">International application No.<br><b>PCT/CN2021/106121</b></td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| A | 周晓平 等 (ZHOU, Xiaoping et al.). "从气象医学理论探析卫气的实质 (From Theory of Meteorological Medicine to Explore the Essential of Ying-Qi and Wei-qi)" 《中国中医基础医学杂志》 ("Chinese Journal of Basic Medicine in Traditional Chinese Medicine"), Vol. 13, No. 4, 25 June 2007 (2007-06-25), entire document | 1-10 |
| A | 李具双 (LI, Jushuang). "营气卫气的体, 象之辨 (Non-official translation: Image Identification: Nutritive Qi (Ying Qi) and Defensive Qi (Wei Qi) Structure)" 《中国中医基础医学杂志》 ("Chinese Journal of Basic Medicine in Traditional Chinese Medicine"), Vol. 22, No. 1, 06 April 2016 (2016-04-06), entire document | 1-10 |

Form PCT/ISA/210 (second sheet) (January 2015)

**17**

**EP 4 179 964 A1**

**INTERNATIONAL SEARCH REPORT**

| International application No. |
|---|
| **PCT/CN2021/106121** |

**Box No. III    Observations where unity of invention is lacking (Continuation of item 3 of first sheet)**

This International Searching Authority found multiple inventions in this international application, as follows:

[1]  Group 1: claims 1-3

[2]  Group 2: claims 4 and 5

[3]  Group 3: claims 6-8

[4]  Group 4: claims 9 and 10

[5]  The claimed group 1 and group 2 do not share a same or corresponding technical feature except measuring the meridian path of a human body. However, measuring the meridian path of a human body is the common knowledge in the field, and is not a special technical feature that contributes over the prior art. The claimed group 1 and group 4 and the claimed group 2 and group 3 do not share a same or corresponding technical feature. Therefore, group 1 and group 2, group 1 and group 4, and group 2 and group 3 do not share a same or corresponding special technical feature, do not have technical relationship, do not belong to a single inventive concept, and thus do not satisfy the requirement of unity of invention, and do not comply with PCT Rule 13.1.

1. ☐  As all required additional search fees were timely paid by the applicant, this international search report covers all searchable claims.

2. ☑  As all searchable claims could be searched without effort justifying additional fees, this Authority did not invite payment of additional fees.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on Protest**    ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA/210 (continuation of first sheet) (January 2015)

18

**INTERNATIONAL SEARCH REPORT**
Information on patent family members

International application No.

**PCT/CN2021/106121**

| Patent document cited in search report | | | Publication date (day/month/year) | Patent family member(s) | | | Publication date (day/month/year) |
|---|---|---|---|---|---|---|---|
| CN | 105342565 | A | 24 February 2016 | None | | | |
| CN | 108652595 | A | 16 October 2018 | CN | 108652595 | B | 11 May 2021 |
| CN | 111265405 | A | 12 June 2020 | None | | | |
| CN | 108871609 | A | 23 November 2018 | US | 2018028072 | A1 | 01 February 2018 |
| CN | 111887808 | A | 06 November 2020 | None | | | |
| WO | 2009158161 | A2 | 30 December 2009 | US | 2009326381 | A1 | 31 December 2009 |

Form PCT/ISA/210 (patent family annex) (January 2015)